(19) Europäisches Patentamt
European Patent Office
Office européen des brevets

(11) EP 1 156 067 B1

(12) EUROPEAN PATENT SPECIFICATION

(45) Date of publication and mention of the grant of the patent:
**05.12.2007 Bulletin 2007/49**

(51) Int Cl.:
***C08F 220/28*** *(2006.01)* ***C08F 220/34*** *(2006.01)*
***C08F 220/60*** *(2006.01)* ***A61M 1/34*** *(2006.01)*
***C08L 33/14*** *(2006.01)*

(21) Application number: **01111835.3**

(22) Date of filing: **16.05.2001**

# (54) Blood filter with (meth)acrylic copolymers comprising glycolether and aminoalkyl units

Blutfilter mit (Meth)Acrylcopolymerisate enthaltend Glykolether- und Aminoalkyl-Einheiten

Filtre à sang comprenant des copolymères (meth)acrylique contenant des segments de glycoléther et aminoalcoyle

(84) Designated Contracting States:
**AT BE CH CY DE DK ES FI FR GB GR IE IT LI LU MC NL PT SE TR**

(30) Priority: **17.05.2000 JP 2000145386**

(43) Date of publication of application:
**21.11.2001 Bulletin 2001/47**

(73) Proprietor: **TERUMO KABUSHIKI KAISHA**
**Tokyo (JP)**

(72) Inventors:
- **Tanaka, Masaru**
**Sapporo-shi,**
**Hokkaido (JP)**
- **Tokunaga, Norifumi**
**Tokyo (JP)**

(74) Representative: **TBK-Patent**
**Bavariaring 4-6**
**80336 München (DE)**

(56) References cited:
**EP-A- 0 420 765** **EP-A- 0 469 196**
**WO-A-84/00621** **GB-A- 1 198 052**
**US-A- 4 218 554** **US-A- 4 981 936**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 156 067 B1

## Description

FIELD OF THE INVENTION

The present invention relates to a specified copolymer and a blood filter with using thereof. More particularly, the present invention relates to a blood filter material surface treating material that selectively captures leucocytes and platelets and passes erythrocytes therethrough and that could minimize damages the blood components would receive at the time of filtering.

BACKGROUND OF THE INVENTION

Recently, in the field of blood transfusion, blood preparations free of leucocytes have been used to prevent graft-versus-host diseases (GVHD) and fever. The methods of removing leucocytes are roughly classified into two methods, i.e., a method that filters leucocytes through a fibrous or spongy filter and a method that separates leucocytes from other blood components using a difference in relative density by using a centrifuge. Further, the method of removing leucocytes using a filter includes a method of simultaneously removing platelets and leucocytes and a method of removing leucocytes without accompanying removal of platelets. The former is used mainly in making erythrocyte preparations and the latter is used mainly in making platelet preparations.

As examples of the latter filter, there have been proposed leucocyte removing filer for purifying platelets, comprising a filter having coated on the surface thereof polyalkoxy (meth)acrylate copolymer (Japanese Patent Application Laid-open No. Hei 5-262656) and leucocyte selectively removing filter containing a nonionic hydrophilic group and a basic nitrogen containing functional group (Japanese Examined Patent Publication No. Hei 6-51060).

Besides side effects by leucocytes, those side effects that would be considered to be attributable to activation of coagulation system or complement system at the time of blood filtration have been concerned about. For example, during blood filtration, there may be sometimes observed anaphylaxis symptoms such as blood pressure depression, shock symptoms, and dizziness that would be considered to be due to an increase in blood bradykinin level (Takahashi et al., Transfusion, No. 35, p.967 (1995)). Therefore, it has been considered necessary to prevent the activation of blood (coagulation system, complement system, leucocyte system, and platelet system) that would cause the above-described side effects.

That is, when blood components contact the surface of the material, nonspecific adsorption, denaturation and multi-layer adsorption of proteins in the plasma occur and sticking of platelets and activation of coagulation system, complement system and leucocyte system occur. For example, production of bradykinin, a typical example of activation of coagulation system, is known to be triggered by contact activation such as adsorption of coagulation XII factor, which is a plasma protein, on the surface of the filter, denaturation and the like. Therefore, if the damages of blood components at the time of filtration could be prevented, then side effects upon blood transfusion would be decreased.

To prevent such an activation of blood, there has been proposed a leucocyte removing filter that is surface-treated with a copolymer comprising as a main component 2-hydroxylethyl methacrylate (HEMA), which is considered to cause less activation of blood components at the time of filtration (Japanese Patent Application Laid-open No. Hei 5-194243). However, this filter is a blood filter that passes platelets and selectively captures leucocytes. Although reportedly it causes less activation, it cannot be said that the activation of complements is prevented sufficiently.

Furthermore, filters with a surface composed of a homopolymer of a quaternary amine only have high leucocyte removing ability. However, because of their high cation density, they show high degrees of nonspecific adsorption of erythrocyte and plasma proteins so that cells that adhered on the surface of the material show considerable activation (cf. Kataoka et al., Biomaterial, Corona, p. 152 (1999)). This causes problems such as a decrease in filtration rate and increased damage of blood components.

GB-B-1,198,052 discloses a process of making stable dispersions of synthetic polymer particles in an inert organic liquid by dispersing preformed particles of the polymer in the liquid in the presence of a stabiliser containing polar groups, the dispersed polymer containing polar groups capable of entering into strong specific interaction with polar groups in the stabiliser so as to cause the stabiliser to be bonded to the polymer particles and provide around them a steric barrier of at least 12 A.

U.S. Patent No. 4,981,936 refers to water soluble terpolymers based on an acrylamide or acrylamide derivative, a cationic vinyl monomer and a hydrophilic vinyl monomer of the formula $CH_2C(R)C(O)O(CH_2(CH_2)_mO)_nE$ where R and E are each hydrogen or alkyl, m is an integer from 1 to 3 and n is an integer from 1 to 25.

EP-A-0 420 765 describes a blood plasma-separating membrane formed of a porous membrane possessing a wetting time in the range of 3 to 500 seconds relative to water and a blood plasma separator using the same.

WO-A-84/00621 is concerned with vinyl acetate copolymers which comprise recurring polymerized units of: 20 to 85 weight percent vinyl acetate monomer, 5 to 65 weight percent acrylate or methacrylate monomer, 5 to 50 weight percent methacrylic acid monomer, and 0.5 to 15 weight percent cationically charged vinyl monomer.

As described above, a blood filter that can remove platelets and leucocytes simultaneously and prevent the activation of blood has been desired.

SUMMARY OF THE INVENTION

Accordingly, an object of the present invention is to provide a novel blood filter unit having high leucocyte and platelet removing ability.

Another object of the present invention is to provide a surface treating agent for blood filters that have obviated problems of activation of blood components at the time of filtration, which is the disadvantage of the conventional blood filters. That is, the present invention provides a blood filter unit which is excellent in blood compatibility and has good wettability with blood, filtration time property or erythrocyte recovery ratio. The present invention provides a blood filter unit having substantially excellent storage stability of blood preparations after filtration.

As described in Japanese Patent Application Laid-open Nos. Hei 5-262656 and Hei 4-152952, generally polyalkoxyalkyl (meth)acrylate and its copolymers are known as materials for medical use having high biocompatibility and antithrombotic activity. That is, when blood is contacted with polyalkoxyalkyl (meth)acrylate and its copolymers, it has been considered that the activation of blood thereby is low and platelets are difficult to be adsorbed thereon. Therefore, no one has conceived of adsorbing platelets with polyalkoxyalkyl (meth)acrylates.

This time the inventors of the present invention have made extensive research with a view to developing blood filter materials that can simultaneously remove leucocytes and platelets while preventing activation of blood. As a result, they have found that the above-described objects can be achieved by use of a copolymer of alkoxyalkyl (meth)acrylate having excellent blood compatibility and an alkylamine ester of acrylic acid having a basic functional group showing low coagulation activity as the site compatible to platelets and leucocytes on the surface of the filter material or as a surface treating agent.

According to the present invention, there is provided a blood filter comprising a filter material surface treated with a copolymer, comprising as constituents an alkoxyalkyl (meth)acrylate represented by the following formula A and at least one comonomer selected from the group consisting of copolymers represented by formulae B, C, D and E.

(A) 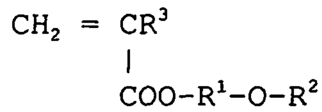

(wherein $R^1$ is an alkylene group having 1 to 4 carbon atoms, $R^2$ is an alkyl group having 1 to 4 carbon atoms, and $R^3$ independently represents hydrogen or a methyl group in each formula)

(B) 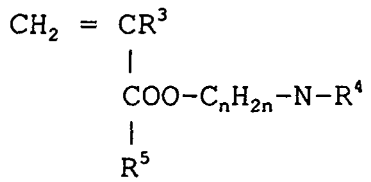

(wherein $R^4$ and $R^5$ independently represent hydrogen or an alkyl group having 1 to 4 carbon atoms in each formula, n is an integer of 1 to 4 in each formula, and $R^3$ is as defined above)

(C) 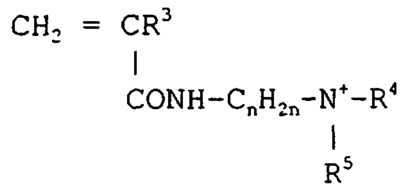

(wherein $R^3$, $R^4$, $R^5$ and n are as defined above)

(D) 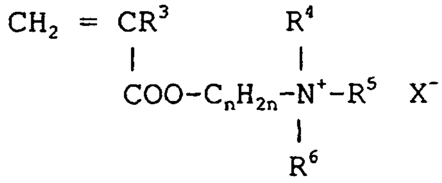

(wherein $R^3$ and $R^4$ are as defined above, R6 independently represents hydrogen or an alkyl group having 1 to 4 carbon atoms in each formula, n is as defined above, and X-independently represents a halogen ion, sulfonic ion or hydrogen sulfate ion)

(E) 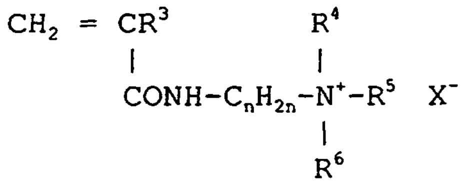

($R^3$, $R^4$, $R^5$ and $R^6$ are as defined above, n is as defined above, and $X^-$ is as defined above).

Here, the "alkyl group having 1 to 4 carbon atoms" refers to a methyl group, an ethyl group, a propyl group, an isopropyl group, a n-butyl group, an isobutyl group or t-butyl group, n is an integer of 1 to 4, preferably an integer of 1 to 3.

The copolymer has the ability of adsorbing leucocytes and platelets and has the activity of preventing the activation of blood. The blood filter material surface-treated with the copolymer has high ability of removing leucocytes and high ability of removing platelets and can prevent the production of bradykinin.

BRIEF DESCRIPTION OF THE INVENTION

Fig. 1 is the cross sectional view of the filter unit of the present invention.

DETAILED DESCRIPTION OF THE INVENTION

The surface of a filter material as used herein refers both to surfaces of the filter material that contact blood to be treated and/or surface portions of pores in the filter material.

The copolymer is a copolymer of one or more monomer of alkoxyalkyl (meth)acrylates represented by the following formula (A) and a comonomer having a basic functional group that is copolymerizable with the monomer.

The alkoxyalkyl (meth)acrylates include methoxymethyl (meth)acrylate, 2-methoxyethyl (meth)acrylate, 2-methoxypropyl (meth)acrylate, methoxybutyl (meth)acrylate, ethoxymethyl (meth)acrylate, ethoxyethyl (meth)acrylate, ethoxypropyl (meth)acrylate, ethoxybutyl (meth)acrylate, propoxymethyl (meth)acrylate, propoxyethyl (meth)acrylate, propoxypropyl (meth)acrylate, propoxybutyl (meth)acrylate, and the like. Here, "(meth)acrylate" stands for acrylate and methacrylate. Among the above monomers, methoxyalkyl (meth)acrylates are preferred from the viewpoints of economy and ease of manipulation. In particular, 2-methoxyethyl (meth)acrylate is preferred.

With regard to the comonomers having a basic functional group that can copolymerize with alkoxyalkyl (meth) acrylates, examples of the basic functional group include primary amino groups, secondary amino groups, tertiary amino groups, quaternary ammonium salts, a pyridyl group, an aziridine group, and an imidazolyl group. Specific comonomers (copolymerizable monomers) of the functional group include the following ones.

Formula B represents aminoalkyl (meth)acrylates. Specific examples thereof include, for example, (meth)acrylic acid esters such as aminomethyl (meth)acrylate, aminoethyl (meth)acrylate, aminoisopropyl (meth)acrylate, amino-n-butyl (meth)acrylate, N-methylaminoethyl (meth)acrylate, N-ethylaminoisobutyl (meth)acrylate, N-isopropylaminomethyl (meth)acrylate, N-isopropylaminoethyl (meth)acrylate, N-n-butylaminoethyl (meth)acrylate, N-t-butylaminoethyl (meth) acrylate, N,N-dimethylaminomethyl (meth)acrylate, N,N-dimethylaminoethyl (meth)acrylate, N,N-dimethylaminopropyl

(meth)acrylate, N,N-dimethylaminobutyl (meth)acrylate, N-methyl-N-ethylaminoethyl (meth)acrylate, N-methyl-N-butylaminoethyl acrylate, N,N-diethylaminoethyl (meth)acrylate, N,N-diethylaminopropyl (meth)acrylate, N,N-dipropylaminoethyl (meth)acrylate, N,N-dipropylaminopropyl (meth)acrylate, and N,N-diaminobutylpropyl (meth)acrylate.

Formula C represents aminoalkyl (meth)acrylamide. Specific examples thereof include, for example, aminomethyl (meth)acrylamide, aminoethyl (meth)acrylamide, aminoisopropyl (meth)acrylamide, amino-n-butyl (meth)acrylamide, N-methylaminoethyl (meth)acrylamide, N-ethylaminoisobutyl (meth)acrylamide, N-isopropylaminomethyl (meth) acrylamide, N-isopropylaminoethyl (meth)acrylamide, N-n-butylaminoethyl (meth)acrylamide, N-t-butylaminoethyl (meth)acrylamide, N,N-dimethylaminomethyl (meth)acrylamide, N,N-dimethylaminoethyl (meth)acrylamide, N,N-dimethylaminopropyl (meth)acrylamide, N,N-dimethylaminobutyl (meth)acrylamide, N-methyl-N-ethylaminoethyl (meth) acrylamide, N-methyl-N-butylaminoethyl acrylamide, N,N-diethylaminoethyl (meth)acrylamide, N,N-diethylaminopropyl (meth)acrylamide, N,N-dipropylaminoethyl (meth)acrylamide, N,N-dipropylaminopropyl (meth)acrylamide, N,N-diaminobutylpropyl (meth)acrylamide and the like.

Formula D and formula E represent each derivatives and the like obtained by treating the compounds of formula B and formula C with an alkyl halide, an alkyl sulfate or the like to convert them into quaternary ammonium salts.

Particularly preferred comonomers among the compounds described above are N,N-dialkylaminopropyl (meth) acrylamides corresponding to formula C in which n is 3, which are easy to synthesize on an industrial scale at low costs, and more specifically N,N-dimethylaminopropyl methacrylamide or N,N-dimethylaminopropyl acrylamide. Also, mention may be made of N,N-dimethylaminoethyl (meth)acrylate, N,N-dimethylaminopropyl (meth)acrylate and N,N-diethylaminoethyl (meth)acrylate corresponding to formula B in which n is 3. However, the comonomer having a basic functional group that can be used in the present invention is not limited to the above-exemplified monomers. The monomer having a basic functional group may be used singly or two or more monomers may be used in combination.

These comonomers are used in proportions to alkoxyalkyl (meth)acrylates such that the compatibility of the polymer to be obtained to blood is not deteriorated. In the copolymer of the present invention, the alkoxyalkyl (meth) acrylate is contained in a proportion of 90 to 10% by mole, preferably 80 to 20% by mole, more preferably 60 to 40% by mole. If the proportion of the alkoxyalkyl (meth)acrylate contained exceeds 90% by mole, the capturing ratio of platelets is too low. On the other hand, if it is below 10% by mole, water solubility increases too much to cause problems in safety and damages to membranes of blood cells are aggravated and the possibility of hemolysis is accompanied so that such copolymers cannot be used as they are.

The copolymers of the present invention may have a molecular weight of several thousands to several hundreds thousands, preferably 5,000 to 500,000. They may be any of random copolymers, block copolymers and graft copolymers. Copolymerization reaction for producing the copolymers is in itself not particularly limited and known methods such as radical polymerization, ion polymerization, photo polymerization, and polymerization using macromers can be used. Various copolymers thus produced are insoluble in water and when in use as a surface treating agent for filters, any one of the copolymers of the present invention may be used singly or plural copolymers may be used in admixture.

The blood filter unit of the present invention is a filter unit used for removing leucocytes and platelets from fluid containing platelets and leucocytes and is used for whole blood, human erythrocyte concentrate (MAP), blood plasma and any other leucocytes-containing and/or platelets-containing suspensions the like in the preparation of blood preparations such as erythrocyte preparations and plasma preparations, or in the therapy using the same. The blood filter unit of the present invention can be applied to cell separation filters such as filters for collecting hematopoietic stem cell and filters for recovering platelets.

The blood filter unit of the present invention is a housing 2 which has at least an inlet tube 7 and an outlet tube 8 and within which a filter material surface-treated with any of the specific copolymers of the present invention as mentioned above is provided. The blood filter unit of the present invention is used to remove leucocytes and platelets from a suspension containing platelets and leucocytes.

In a preferred embodiment as shown in Fig. 1, the housing 2 comprises a base 22 and a cover 21, which are engaged with each other on their lateral sides thereof. The filter material surface-treated with the copolymer of the present invention is held within the housing 2. Between the housing 2 and the filter material are spaced apart a base support 4 and a cover support 41 in such a manner that the supports 4 and 41 in the housing can provide a suitable liquid flow. The filter material is preferably composed of a two-layered carrier including the main filter material 6 which is surface-treated with the copolymer of the present invention and is located on the downstream side, and a prefilter material 10 which is a coarse carrier located on the upstream side to remove impurities and the like.

Exemplary types of the filter material for use in the blood filter unit of the present invention include nonwoven fabric, woven fabric, porous material and beads, and the filter material may be formed into a membrane such as porous flat membrane or hollow fiber membrane, a sheet, a tube or other shapes.

The material used for the filter material is not particularly limited and includes natural polymers such as cotton and hemp celluloses and derivatives thereof, synthetic polymer materials such as nylon, polyolefin, halogenated polyolefin, polyethylene terephthalate, polybutylene terephthalate, polyvinylidene fluoride, polyamide, polyimide, polyurethane, polyester, polysulfone, polyethersulfone, poly(meth)acrylate, ethylene-polyvinyl alcohol copolymer, polyacrylonitrile and butadiene-acrylonitrile copolymer, and mixtures thereof. Polyurethane, polysulfone and polyethersulfone are preferably used for porous material and polyethylene terephthalate and polybutylene terephthalate are preferably used for nonwoven fabric.

In the case of nonwoven fabric, the filament used may be a monofilament or a multifilament, or a porous filament or a deformed filament.

In the case of porous material, the average pore size is in the range of from 1 μm ($1\times10^3$ nm) to 20 μm ($20\times10^3$ nm). If the average pore size is 1 μm or less, the filter tends to be clogged while if it is above 20 μm, the removal rate of leucocytes or platelets decreases to 50% or less.

When nonwoven fabric is used for the filter material, the nonwoven fabric has preferably an average fiber diameter of not more than 100 μm. If the fiber diameter exceeds this value, it is difficult for the base material to have a sufficient surface area for filtration.

When porous beads are used for the filter material, the porous beads have preferably an average particle size of 25 μm to 300 μm. If the average particle size is less than 25 μm, filtration pressure is increased, whereas if the average particle size exceeds 300 μm, sufficiently high filtration efficiency cannot be obtained due to decrease of the surface area per volume.

The amount of the surface treating agent carried on the filter material is preferably in the range of from 0.1 to 50 mg/g, preferably 0.3 to 30 mg/g.

The object of the present invention can be achieved by having the copolymer of the present invention carried on the surface of the blood filter described above.

Alternatively, the blood filter material of the present invention may be obtained by being carried on the copolymer which is obtained by the copolymerizing the alkoxyalkyl (meth)acrylate represented by formula A with at least one copolymer selected from the group consisting of aminostyrene, N,N-dimethylaminostyrene, N,N-diethylaminostyrene, vinylpyridine, N-methyl-N-vinylpyridine, N-ethyl-N-vinylpyridine, vinylquinoline, ethyleneimine, propyleneimine, N-aminoethylethyleneimine, vinylimidazole, vinylpyrazoline, and vinylpyrazine. Either the filter material of the present invention may be carried on the another copolymer obtained by reacting the resulting copolymer with an alkyl halide, an alkyl sulfate or the like to convert them into quaternary ammonium salts thereof.

The method for holding the copolymer on the surface of a filter material includes known methods such as a coating method, a graft copolymer using radioactive rays, electron beam and ultraviolet rays, and a method of introducing the copolymer using chemical reaction with functional groups in the base material. Of these, the coating method is practically preferable since the production step is easy to perform. The coating method includes an applying method, a spraying method, a dipping method and the like but is not particularly limited and any of them can be applied.

For example, the coating treatment by the method of applying the copolymer can be practiced by simple operations such as dipping a filter material in a coating solution having dissolved the copolymer in a suitable organic solvent such as alcohols, chloroform, acetone, tetrahydrofuran or dimethylformamide, removing excessive solution and then air drying. To more firmly fix the copolymer to the filter material, the filter material after the coating may be heated to further increase the adhesion between the filter material and the copolymer.

The blood filter material having the copolymer fixed on the surface thereof exhibits high removal rates for leucocytes and platelets, respectively, but shows less activation of blood components such as an increase in blood bradykinin so that it does not deteriorate the quality of blood after the filtration. The copolymer can easily control the adsorbability of leucocytes and platelets by suitably changing the composition and ratio of comonomer having a basic functional group. The copolymer contains alkoxyalkyl (meth)acrylate having excellent compatibility with blood as a component of the copolymer so that it has excellent wettability with blood and can realize a high bleeding rate and filtration rate. Further, the filter unit of the invention has a high erythrocyte recovery rate and causes no hemolysis after the filtration so that it can exhibit excellent long term storage stability of blood.

The removal rate of leucocytes in human erythrocyte concentrated solution by use of the filter unit of the present invention is 99% or more, particularly 99.5% or more. The platelet removal rate is 99% or more.

Since the copolymer in itself is a material excellent in compatibility with blood, it can be used not only as a blood filter but also as a surface modifier for various medical apparatuses and tools such as blood storage bag, blood circuit, indwelling needle, catheter, guide wire, stent, oxygenator, and dialyzer.

## EXAMPLES

Hereinafter, the present invention will be described in detail by examples. However, the present invention is not limited thereto. Examples 1 to 11 relate to production of surface treating agents and Example 12 and Comparative Examples 1 and 2 relate to characteristic tests of blood filter units.

(Example 1)

To 20 g of 2-methoxyethyl acrylate (MEA) (Osaka Organic Chemistry) and 10.3 g of dimethylaminopropylacrylamide (Kojin) was added azobisisobutyronitrile (radical polymerization initiator) (Tokyo Kasei) in an amount of 0.2% by weight based on the total weight of monomers and the mixture was subjected to polymerization in 120 g of 1,4-dioxane (Kanto Chemical) at 80°C for 8 hours. After completion of the polymerization, the reaction mixture was dripped into n-hexane (Kanto Chemical) to form precipitates and the product was isolated. The product was dissolved in acetone (Kanto Chemical) and the solution was dripped into n-hexane and thus purified twice. The purified product was dried under reduced pressure over a whole day. The amine composition (mol%) of the obtained polymer was obtained by $^1H$-NMR. This was named surface treating agent 1 (MEA : DMAPAAm = 80 : 20).

(Example 2)

The same procedures were repeated as in Example 1 except that 15 g of 2-methoxyethyl acrylate (MEA) and 12 g of dimethylaminopropylacrylamide (DMAPAAm) were used as the starting materials to obtain surface treating agent 2 (MEA : DMAPAAm = 60 : 40).

(Example 3)

The same procedures were repeated as in Example 1 except that 20 g of 2-methoxyethyl acrylate (MEA) and 6.5 g of dimethylaminopropylacrylamide (DMAPMAAm) (Aldrich) were used as the starting materials to obtain surface treating agent 3 (MEA : DMAPMAAm = 80 : 20).

(Example 4)

The same procedures were repeated as in Example 1 except that 15 g of 2-methoxyethyl acrylate (MEA) and 13.1 g of dimethylaminopropylacrylamide (DMAPMAAm) were used as the starting materials to obtain surface treating agent 2 (MEA : DMAPMAAm = 60 : 40).

(Example 5)

To 20 g of 2-methoxyethyl acrylate (MEA) and 6 g of dimethylaminoethyl methacrylate (DMAEMA) (Tokyo Kasei) was added in an amount of 0.1% by weight based on the total weight of monomers and the mixture was subjected to polymerization in 120 g of (dimethylformamide) DMF (Kanto Chemical) at 75°C for 8 hours. After completion of the polymerization, the reaction mixture was dripped into n-hexane (Kanto Chemical) to form precipitates and the product was isolated. The product was dissolved in tetrahydrofuran and the solution was dripped into n-hexane and thus purified twice. The purified product was dried under reduced pressure over a whole day. This was named surface treating agent 1 (MEA : DMAEMA = 80 : 20).

(Example 6)

The same procedures were repeated as in Example 5 except that 17 g of 2-methoxyethyl acrylate (MEA) and 13.7 g of dimethylaminoethyl methacrylate (DMAEMA) were used as the starting materials to obtain surface treating agent 6 (MEA : DMAEMA = 60 : 40).

(Example 7)

The same procedures were repeated as in Example 5 except that 3.3 g of 2-methoxyethyl acrylate (MEA) and 16.0 g of dimethylaminoethyl methacrylate (DMAEMA) were used as the starting materials to obtain surface treating agent 7 (MEA : DMAEMA = 20 : 80).

(Example 8)

The same procedures were repeated as in Example 5 except that 20 g of 2-methoxyethyl acrylate (MEA) and 7.1 g of diethylaminoethyl methacrylate (DEAEMA) (Wako Pure Chemical Industry) were used as the starting materials to obtain surface treating agent 8 (MEA : DEAEMA = 80 : 20).

(Example 9)

The same procedures were repeated as in Example 5 except that 15 g of 2-methoxyethyl acrylate (MEA) and 14.3 g of diethylaminoethyl methacrylate (DEAEMA) were used as the starting materials to obtain surface treating agent 9 (MEA : DEAEMA = 60 : 40).

(Example 10)

The same procedures were repeated as in Example 5 except that 20 g of 2-methoxyethyl acrylate (MEA) and 5.5 g of dimethylaminoethyl acrylate (DMAEA) (Kojin) were used as the starting materials to obtain surface treating agent 10 (MEA : DMAEA = 80 : 20).

(Example 11)

The same procedures were repeated as in Example 5 except that 17 g of 2-methoxyethyl acrylate (MEA) and 12.5 g of dimethylaminoethyl acrylate (DMAEA) (Kojin Co., Ltd.) were used as the starting materials to obtain surface treating agent 11 (MEA : DMAEA = 60 : 40).

(Example 12)

The surface treating agents prepared in Examples 1 to 11 were each dissolved in methanol and each of the solutions was coated on a urethane porous material (Nippon Miractorane E394 POTA, maximum pore size: 10 μm, porosity: 85%) and then washed by showering with warm water at 60°C. After drying it, the resulting blood filter material was punched to pieces of a size of 0.6 mm in thickness and 55 mm in diameter. These were assembled in a blood circuit and MAP (human erythrocyte concentrate) was treated in the blood circuit.

Weights of blood before and after filtration, concentration of leucocytes, and concentration of platelets were calculated using automatic blood cell counter (Sysmex NE-6000, produced by Toa Medical Electronics) and then leucocyte removal ratio and platelet removal ratio were obtained.

Leucocyte removal ratio = (1 - (number of leucocytes after filtration)/(number of leucocytes before filtration)) × 100

Platelet removal ratio = (1 - (number of platelets after filtration)/(number of platelets before filtration)) × 100

The production amount of bradykinin was determined by sampling blood at the time of filtering the blood. Upon measurement, 5 mM 1,1-phenanthroline (Tokyo Kasei) as a bradykinin decomposition inhibitor was added. Table 1 shows relationships of leucocyte removal ratio, platelet removal ratio and bradykinin production amount.

When blood was filtered, the time in which the blood contacted the filter and bled through it was also measured. Table 2 shows the results. As for the blood bleeding rate, a conventional blood filter composed mainly of HEMA was also tested for comparison.

(Comparative Example 1)

Non-treated urethane made porous material without coating was attached to a blood circuit similar to that used in Example 11 and MAP was filtered therewith and leucocyte removal ratio, platelet recovery ratio and bradykinin production amount were obtained. Table 1 shows the results.

(Comparative Example 2)

Homopolymer consisting of poly(2-methoxyethyl acrylate) (PMEA) as a coating polymer was coated on a urethane made porous material in the same manner as in Example 12 to obtain a blood filter and MAP was filtered in a blood circuit therethrough and leucocyte removal ratio, platelet recovery ratio, and ratio of bradykinin production amount to that of non-treated film were obtained. Table 1 shows the results.

Table 1

| Surface treating agent | Leucocyte removal ratio (%) | Platelet removal ratio (%) | Bradykinin production amount (vs. non-treated one) |
|---|---|---|---|
| 1 (MEA:DMAPAAm=80:20) | 99.7 | 99.4 | <1/10 |
| 2 (MEA:DMAPAAm=60:40) | 99.8 | 99.9 | <1/10 |
| 3 (MEA:DMAPMAAm=80: 20) | 99.8 | 99.7 | <1/10 |
| 4 (MEA:DMAPMAAm=60: 40) | 99.9 | 99.9 | <1/10 |
| 5 (MEA:DMAEMA=80:20) | 99.9 | 99.9 | <1/10 |
| 6 (MEA:DMAEMA=60:40) | 99.9 | 100 | <1/10 |
| 7 (MEA:DMAEMA=20:80) | 99.9 | 100 | <1/10 |
| 8 (MEA:DAEMA=80:20) | 99.8 | 99.9 | - (No measured value) |
| 9 (MEA:DEAEMA=80:20) | 99.7 | 99.3 | <1/10 |
| 10 (MEA:DMAEA=80:20) | 99.8 | 99.9 | <1/10 |
| Non-treated (Comparative Example 1) | 87.2 | 18.1 | 1 |
| PEMA (Comparative Example 2) | 93.6 | 3.7 | 1/3 |

As will be apparent from Table 1, all the surface treating agents in the above Examples exhibited excellent leucocyte and platelet removing abilities. As for the production amount of bradykinin at the time of filtration, the blood filter material of the invention had a value as low as about 1/10 time or less that of the non-treated filter material of Comparative Example 1. In Comparative Example 2, the production amount of bradykinin at the time of filtration decreased to about 1/3 time or less of that of Comparative Example 1 but the platelet removal ratio was as low as 3.7%.

Table 2, Time of bleeding of blood

| Surface treating agent | Time (second) |
|---|---|
| Example 1: (MEA:DMAPAAm=80:20) | 15 |
| Example 2: (MEA:DMAPAAm=60:40) | 17 |
| Example 3: (MEA:DMAPMAAm=80:20) | 14 |
| Example 4: (MEA:DMAPMAAm=60:40) | 19 |
| Example 5: (MEA:DMAEMA=80:20) | 20 |
| Example 6: (MEA:DMAEMA=60:40) | 22 |
| Example 7: (MEA:DMAEMA=20:80) | 25 |
| Example 8: (MEA:DEAEMA =80:20) | 19 |
| Example 9: (MEA:DEAEMA=60:40) | 22 |

(continued)

| Surface treating agent | Time (second) |
|---|---|
| Example 10: (MEA:DMAEA=80:20) | 14 |
| Example 11: (MEA:DMAEA=60:40) | 20 |
| Comparative Example 1: Non-treated | 32 |
| Comparative Example 2: PMEA | 13 |

Use of a surface treating agent excellent in blood compatibility as a filter material for removing leucocytes enable one to efficiently remove leucocytes and platelets while suppressing the activation of blood components at the time of filtration to a low level so that effective means are provided in the field of safe and high quality blood preparations and leucocyte removal therapy against autoimmune diseases. The present invention provides a surface treating agent that is very easy to produce and can be applied to blood compatible materials for medical apparatuses.

As described above, introduction of the copolymer in at least a surface portion of the filter material can impart the filter with the above-described blood compatibility to decrease the activation of blood components at the time of filtration. Further, excellent affinity for blood leads to an increase in the characteristic of bleeding of blood through the filter so that filtration time can be shortened. If blood is retained in the filter at the time of filtration, an increased amount of bradykinin is produced. Hence, shortened filtration time will be effective in decreasing the production of bradykinin.

A copolymer having excellent blood compatibility comprising an alkoxyalkyl (meth)acrylate and a comonomer having a basic functional group, a surface treating agent for blood filter using the same, and blood filter coated with the same on the surface thereof. The copolymer is useful as blood filter material for efficiently removing leucocytes and platelets while preventing damages of blood components at the time of blood filtration to a low level.

**Claims**

1. A blood filter comprising a filter material surface treated with a copolymer for filtering blood, wherein said copolymer comprises a monomer represented by formula A and at least one comonomer selected from the group consisting of copolymers represented by formulae B, C, D and E:

(A)

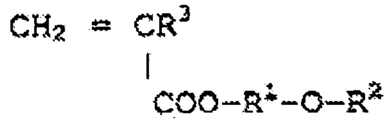

(wherein $R^1$ is an alkylene group having 1 to 4 carbon atoms, $R^2$ is an alkyl group having 1 to 4 carbon atoms, and $R^3$ independently represents hydrogen or a methyl group in each formula);

(B)

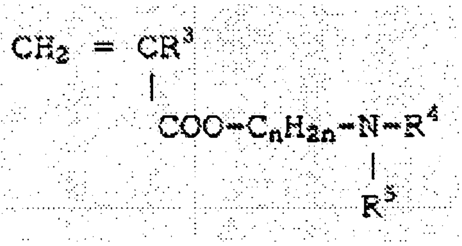

(wherein $R^4$ and $R^5$ independently represents hydrogen or an alkyl group having 1 to 4 carbon atoms in each formula, n is an integer in each formula, and $R^3$ is as defined above)

(C) 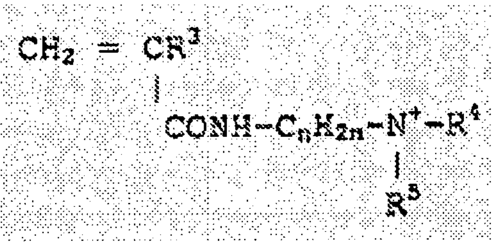

(wherein $R^3$, $R^4$, $R^5$ and n are as defined above);

(D) 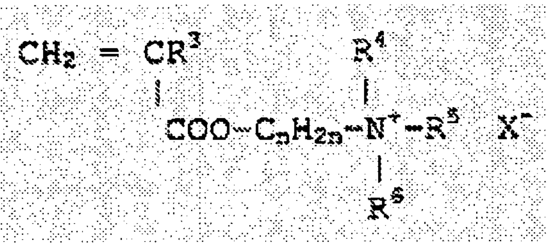

(wherein $R^3$, $R^4$ and $R^5$ are as defined above, $R^6$ independently represents hydrogen or an alkyl group having 1 to 4 carbon atoms in each formula, n is as defined above, and $X^-$ independently represents a halogen ion, sulfonic ion or hydrogen sulfate ion);

(E) 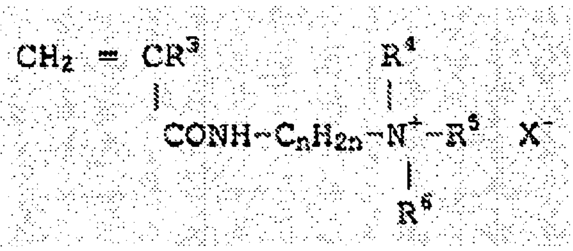

($R^3$, $R^4$, $R^5$, and $R^6$ are as defined above, n is as defined above, and $X^-$ is as defined above).

2. The blood filter according to claim 1, comprising the monomer represented by the formula A in an amount of 10 to 90 mol%.

3. The blood filter according to claim 1 or 2, wherein the monomer represented by the formula A is 2-methoxyethyl (meth)acrylate.

4. The blood filter according to any one of claims 1 to 3, wherein the monomer represented by the formula B is at least one selected from the group consisting of N,N-dimethylaminoethyl (meth)acrylate, N,N-dimethylaminopropyl (meth) acrylate, and N,N-diethylaminoethyl (meth)acrylate.

5. The blood filter according to any one of claims 1 to 4, wherein the monomer represented by the formula C is at least one selected from dimethylaminopropylmethacrylamide and dimethylaminopropylacrylamide.

6. The blood filter according to any one of claims 1 to 5, wherein the filter unit is an adsorbent for leucocytes and platelets.

7. A blood filter unit which comprises a housing having at least an inlet tube and an outlet tube, and a blood filter according to any one of claims 1 to 6 which is located within the housing.

8. The blood filter unit according to claim 7, wherein the blood filter comprises polyurethane or polyester as a main component.

9. Use of the blood filter unit according to claim 7 or 8, for removing leucocytes and platelets from a suspension containing platelets and leucocytes.

**10.** A method of producing a blood filter according to any one of claims 1 to 6, comprising coating the copolymer to a surface of a blood filter material and heat drying it.

## Patentansprüche

**1.** Blutfilter mit einer Filtermaterialoberfläche, die mit einem Copolymer behandelt ist, zum Filtrieren von Blut, wobei das Copolymer ein Monomer, welches durch die Formel A wiedergegeben wird, und wenigstens ein Comonomer ausgewählt aus der Gruppe bestehend aus den Copolymeren, welche durch die Formeln B, C, D und E wiedergegeben werden, umfasst:

(A) 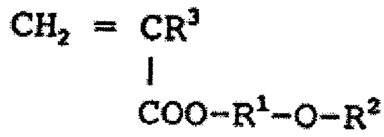

(wobei $R^1$ eine Alkylengruppe mit 1 bis 4 Kohlenstoffatomen ist, $R^2$ eine Alkylgruppe mit 1 bis 4 Kohlenstoffatomen ist und $R^3$ in jeder Formel unabhängig Wasserstoff oder eine Methylgruppe bezeichnet);

(B) 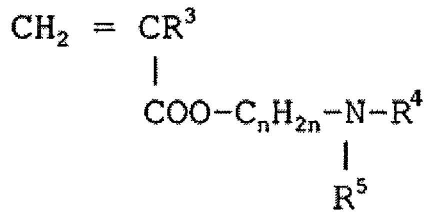

(wobei $R^4$ und $R^5$ in jeder Formel unabhängig Wasserstoff oder eine Alkylgruppe mit 1 bis 4 Kohlenstoffatomen bezeichnen, n in jeder Formel eine ganze Zahl ist und $R^3$ wie vorstehend definiert ist);

(C) 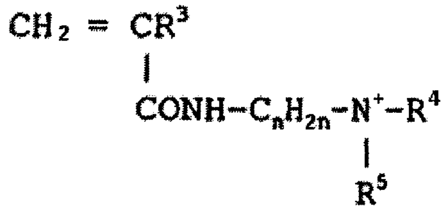

(wobei $R^3$, $R^4$, $R^5$ und n wie vorstehend definiert sind);

(D) 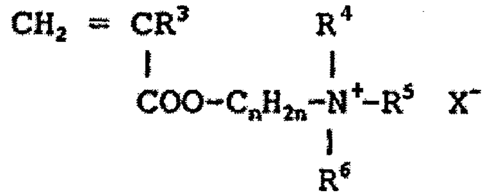

(wobei $R^3$, $R^4$ und $R^5$ wie vorstehend definiert sind, $R^6$ in jeder Formel unabhängig Wasserstoff oder eine Alkylgruppe mit 1 bis 4 Kohlenstoffatomen bezeichnet, n wie vorstehend definiert ist und $X^-$ unabhängig ein Halogenion, ein Sulfonsäureion oder ein Hydrogensulfation bezeichnet);

(E) 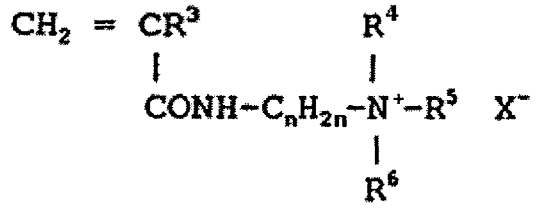

($R^3$, $R^4$, $R^5$ und $R^6$ sind wie vorstehend definiert, n ist wie vorstehend definiert und $X^-$ ist wie vorstehend definiert).

2. Blutfilter nach Anspruch 1, der das durch die Formel A wiedergegebene Monomer in einer Menge von 10 bis 90 mol% umfasst.

3. Blutfilter nach Anspruch 1 oder 2, wobei das durch die Formel A wiedergegebene Monomer 2-Methoxyethyl(meth) acrylat ist.

4. Blutfilter nach einem der Ansprüche 1 bis 3, wobei das durch die Formel B wiedergegebene Monomer wenigstens eines ist ausgewählt aus der Gruppe bestehend aus N,N-Dimethylaminoethyl(meth)acrylat, N,N-Dimethylaminopropyl(meth)acrylat und N,N-Diethylaminoethyl(meth)-acrylat.

5. Blutfilter nach einem der Ansprüche 1 bis 4, wobei das durch die Formel C wiedergegebene Monomer wenigstens eines ist ausgewählt aus Dimethylaminopropylmethacrylamid und Dimethylaminopropylacrylamid.

6. Blutfilter nach einem der Ansprüche 1 bis 5, wobei die Filtereinheit ein Adsorbens für Leukozyten und Blutplättchen ist.

7. Blutfiltereinheit, welche ein Gehäuse mit wenigstens einem Einlassrohr und einem Auslassrohr sowie einen Blutfilter nach einem der Ansprüche 1 bis 6, welcher sich in dem Gehäuse befindet, aufweist.

8. Blutfiltereinheit nach Anspruch 7, wobei der Blutfilter als eine Hauptkomponente Polyurethan oder Polyester umfasst.

9. Verwendung der Blutfiltereinheit nach Anspruch 7 oder 8 zum Entfernen von Leukozyten und Blutplättchen aus einer Suspension, welche Blutplättchen und Leukozyten enthält.

10. Verfahren zur Herstellung eines Blutfilters nach einem der Ansprüche 1 bis 6 mit einem Auftragen des Copolymers auf eine Oberfläche eines Blutfiltermaterials und einem Trocknen von diesem mit Wärme.

## Revendications

1. Filtre à sang comprenant un matériau filtrant traité en surface avec un copolymère adapté à la filtration de sang, lequel copolymère comporte des motifs d'un monomère représenté par la formule A et d'au moins un comonomère choisi parmi les monomères représentés par les formules B, C, D et E :

(A) 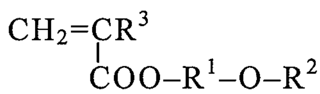

dans laquelle formule $R^1$ représente un groupe alkylène comportant 1 à 4 atomes de carbone, $R^2$ représente un groupe alkyle comportant 1 à 4 atomes de carbone, et $R^3$ représente un atome d'hydrogène ou un groupe méthyle, indépendamment dans chaque formule ;

(B)

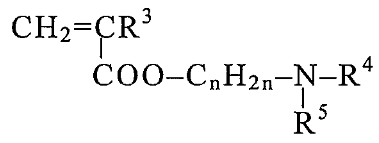

dans laquelle $R^4$ et $R^5$ représentent chacun, indépendamment dans chaque formule, un atome d'hydrogène ou un groupe alkyle comportant 1 à 4 atomes de carbone, n représente un nombre entier dans chaque formule, et $R^3$ a la signification indiquée ci-dessus ;

(C)

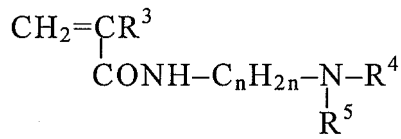

dans laquelle $R^3$, $R^4$, $R^5$ et n ont les significations indiquées ci-dessus ;

(D)

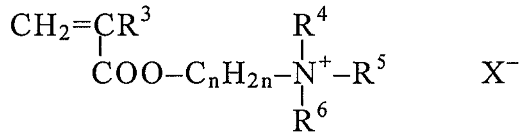

dans laquelle $R^3$, $R^4$ et $R^5$ ont les significations indiquées ci-dessus, $R^6$ représente, indépendamment dans chaque formule, un atome d'hydrogène ou un groupe alkyle comportant 1 à 4 atomes de carbone, n a la signification indiquées ci-dessus, et X- représente un ion halogénure, un ion sulfonate ou un ion hydrogénosulfate ;

(E)

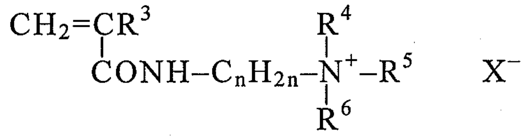

dans laquelle $R^3$, $R^4$, $R^5$ et $R^6$ ont les significations indiquées ci-dessus, n a la signification indiquées ci-dessus, et X- a la signification indiquée ci-dessus.

**2.** Filtre à sang conforme à la revendication 1, dans lequel le monomère représenté par la formule A se trouve en une proportion de 10 à 90 % en moles.

**3.** Filtre à sang conforme à la revendication 1 ou 2, dans lequel le monomère représenté par la formule A est de l'acrylate ou du méthacrylate de 2-méthoxy-éthyle.

**4.** Filtre à sang conforme à l'une des revendications 1 à 3, dans lequel le monomère représenté par la formule B est au moins un monomère choisi dans l'ensemble formé par les acrylate et méthacrylate de N,N-diméthyl-aminoéthyle, acrylate et méthacrylate de N,N-diméthyl-aminopropyle, et acrylate et méthacrylate de N,N-diéthyl-aminoéthyle.

**5.** Filtre à sang conforme à l'une des revendications 1 à 4, dans lequel le monomère représenté par la formule C est au moins un monomère choisi parmi le diméthylaminopropyl-méthacrylamide et le diméthylaminopropyl-acrylamide.

**6.** Filtre à sang conforme à l'une des revendications 1 à 5, dans lequel le matériau filtrant est un adsorbant pour les leucocytes et les plaquettes.

7. Unité de filtre à sang qui comporte un logement muni d'au moins un tube d'entrée et un tube de sortie, et un filtre à sang conforme à l'une des revendications 1 à 6, disposé à l'intérieur du logement.

8. Unité de filtre à sang conforme à la revendication 7, dans laquelle le filtre à sang comporte comme composant principal un polyuréthane ou un polyester.

9. Emploi d'une unité de filtre à sang, conforme à la revendication 7 ou 8, en vue de séparer des leucocytes et des plaquettes d'une suspension contenant des leucocytes et des plaquettes.

10. Procédé de production d'un filtre à sang conforme à l'une des revendications 1 à 6, qui comporte le fait de revêtir du copolymère une surface d'un matériau de filtre à sang et le fait de sécher le copolymère par chauffage.

FIG.1

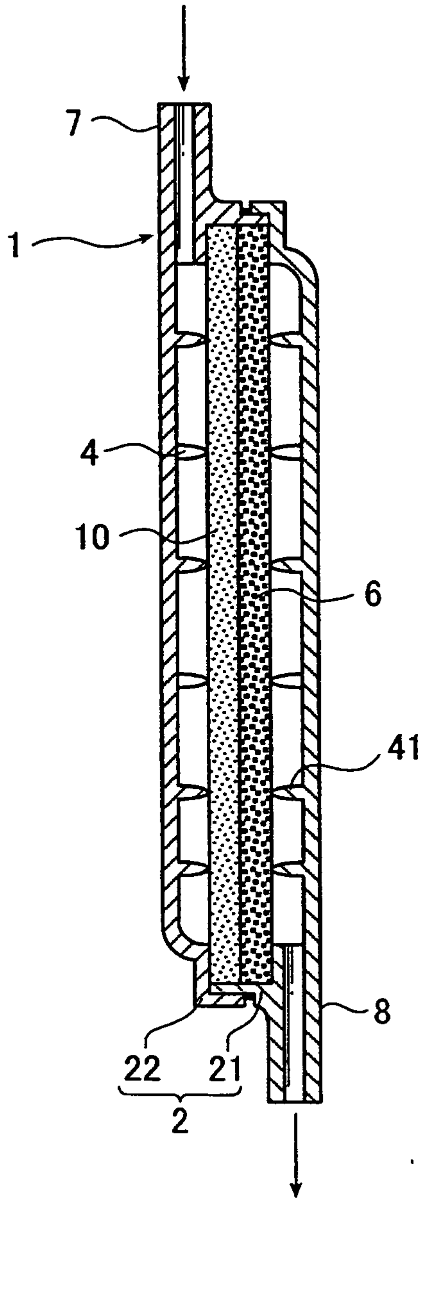

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5262656 A
- JP 6051060 B
- JP 5194243 A
- GB 1198052 B
- US 4981936 A
- EP 0420765 A
- WO 8400621 A
- JP 4152952 A


### Non-patent literature cited in the description


- **TAKAHASHI et al.** *Transfusion,* 1995, vol. 35, 967
- **KATAOKA et al.** *Biomaterial, Corona,* 1999, 152